Europäisches Patentamt

European Patent Office

Office européen des brevets

(11) Publication number: **0 089 152**
**B1**

(12) # EUROPEAN PATENT SPECIFICATION

(45) Date of publication of patent specification: **15.10.86**

(21) Application number: **83301149.7**

(22) Date of filing: **03.03.83**

(51) Int. Cl.⁴: **A 61 L 15/04, C 08 H 1/00, C 08 B 37/08**

(54) **Hydrophilic bipolymeric copolyelectrolytes, and biodegradable wound dressings comprising same.**

(30) Priority: **17.03.82 US 358994**

(43) Date of publication of application:
**21.09.83 Bulletin 83/38**

(45) Publication of the grant of the patent:
**15.10.86 Bulletin 86/42**

(84) Designated Contracting States:
**AT BE CH DE FR GB IT LI LU NL SE**

(56) References cited:
**EP-A-0 038 628**
**FR-A-2 318 189**
**FR-A-2 332 863**
**FR-A-2 432 046**

(73) Proprietor: **University of Illinois Foundation University of Illinois P.O. Box 4348 Chicago Illinois 60680 (US)**

(72) Inventor: **Widra, Abe 307 Keystone Avenue River Forest Illinois 60305 (US)**

(74) Representative: **Allam, Peter Clerk et al LLOYD WISE, TREGEAR & CO. Norman House 105-109 Strand London WC2R 0AE (GB)**

Courier Press, Leamington Spa, England.

## Description

This invention relates to novel hydrophilic biopolymeric materials and, more particularly, to the use of such materials in the form of hydrogel membranes as biodegradable dressings for burn wounds and other denuded tissue wound sites.

In the therapeutic procedure for treating an extensively burned patient, devitalized tissue is removed from the burn site, and the debrided areas are covered with a temporary burn wound dressing prior to definitive autografting. The temporary burn wound dressing ideally provides several important therapeutic functions. First of all, it serves as a barrier to prevent loss of water, salts, and proteins from the internal milieu while blocking microbial infection from the environment. Secondly, it serves to improve wound bed base and promote wound closure, thereby facilitating decontamination and regeneration of the wound area. Thirdly, it serves to alleviate pain.

The best wound coverage material is skin itself — a biologic dressing with a collagenous component rendering it adherent to endogenous fibrin, and a keratinized water proof surface. Biologic dressings in current use include commercially available pigskin heterograft, and living (donor) human or human cadaver homograft material. While human skin has a number of advantages over pigskin for this purpose, there are many problems associated with obtaining, storing, and using frozen and lyophilized human skin. Skin banks require at least 200 cadavers per year in order to supply existing burn centers. In any case, either of these types of biologic dressings give rise to rejection phenomena which mandate their removal and replacement every 2 to 5 days. Stripping after 5 days of adherence often results in bleeding and renewed destruction of the graft base.

The body's rejection of foreign biologic dressings has lead to a search for skin substitutes which are either completely synthetic, derived from tissue components, or some combination thereof. Such search has not heretofore met with great success due to the difficulties involved in finding a material exhibiting the proper combination of properties essential for an ideal skin substitute. These properties include rapid, uniform, and strong adherence to underlying tissues; water vapor transport characteristics sufficient to keep the underlying tissues moist without creating pooling; elasticity; durability; intact bacterial barrier characteristics; nonantigenicity and nontoxicity; high permeability to oxygen; capability of being easily applied and removed; easily storable; and relatively inexpensive.

The materials previously proposed as skin substitutes have generally been found to be lacking in one or more of the foregoing properties. The most satisfactory of these materials have consisted of layered composite membranes having an outer layer designed for durability and elasticity, such as silicone or other synthetic polymeric film; and an inner layer designed for maximum adherence, such as collagen, cotton gauze, or Dacron flocking. However, the necessity for these composite membrane burn wound dressings to be stripped from the wound prior to definitive autografting, poses some difficult design problems. Since these dressings generally depend upon tissue ingrowth into their inner layer for adherence to the wound, complete removal of the dressing is difficult and may require re-debridement before grafting. While it may be possible to overcome this problem by having the inner layer formed of a biodegradable material, such approach has been found to result either in a premature loss of adherence and effectiveness of the dressing before completion of wound healing, or in the formation of considerable scar tissue, unless the biodegradation rate of the inner layer is carefully controlled so as to precisely coincide with the rate of wound healing.

Homogeneous hydrogel membranes formed from the hydrophilic synthetic polymeric material, Hydron, have also been proposed as skin substitutes for use as burn wound dressings. This material combines adherency to dry and moist tissue with the other desirable properties of conformability to regular contour, elasticity, nonantigenicity, being inert, and providing an effective antimicrobial barrier. Its major drawbacks as a burn wound dressing, however, are its excessive permeability to water and its low degree of durability when washed with water or in the presence of moderate oozing or bleeding. Moreover, its elasticity is too rapidly lost upon drying. For these reasons, it has not proven to be practical for relatively long-term burn wound dressing applications.

Other surgical materials which are based on collagen have been proposed. For example, FR—A—2318189 discloses composite materials comprising a polymer of fibrous (insoluble) collagen and a mucopolysaccharide which is crosslinked to render it more resistant to resorption than native collagen. The resulting cross-linked material may be used for surgical sutures, blood vessel grafts and surgical prostheses, for example. In FR—A—2332863, it is disclosed that a synthetic skin may be obtained by forming a multilayer membrane, one layer of which is a crosslinked polymer of the kind taught in FR—A—2318189, *supra;* and the other of which is a non-toxic material, such as a synthetic polymer, which controls the moisture flux of the overall membrane. Again, EP—A—0038628 discloses a composite material of de-N-acetylated chitin and fibrous collagen which may be replaced in part by gelatin and/or soluble collagen. The composite material is useful for a number of surgical purposes, eg as a prosthetic device, where non-biodegradable properties are desired.

## Summary of the Invention

In view of the state of the art, it would be desirable to provide a biodegradable material exhibiting the proper combination of properties rendering it suitable for use as a dressing for burn wounds and other denuded tissue wound sites.

In accordance with the present invention there are provided novel hydrophilic biopolymeric copolyelectrolytes of (a) a water-soluble linear anionic protein polyelectrolyte component derived from keratin and (b) a water-soluble linear cationic biopolymer polyelectrolyte component derived from at least one biopolymer selected from the group consisting of a glucosaminoglycan and collagen.

Hydrogel membranes formed from the copolyelectrolytes of the present invention exhibit a combination of properties rendering them useful as biodegradable dressings for burn wounds and other denuded tissue wound sites. Such membranes are strongly adherent to underlying tissues, elastic, durable, highly permeable to oxygen, absorbent to wound exudates without losing their durability, have water vapor transport characteristics sufficient to keep the underlying tissues moist without creating pooling, and have intact bacterial barrier characteristics. They may be readily and conveniently applied to the wound site in several alternative modes. By virtue of their biodegradability, they do not require stripping. In the latter stages of wound healing, when moisture through the wound area is much reduced, any remaining copolyelectrolyte material will dry and harden to a protective carapace, which will fall off naturally without leaving any scarring.

Description of Preferred Embodiments

The hydrophilic biopolymeric copolyelectrolytes of the present invention are water-insoluble, water-swellable materials prepared from water-soluble derivatives of the protein, keratin, and of at least one other biopolymer selected from a glucosaminoglycan, such as chitosan, and the protein, collagen. Keratin is a protein obtained from sources such as skin, fur, hair, wool, horn, nails, claws, beaks and scales. It may be readily isolated from its source material and separated into its alpha-keratose and gamma-keratose fractions by procedures well known in the art, such as, for example, as described by Widra, Mycopathologia et Mycologia Applicata, Volume 30, pages 141—144 (1966) and Rhodes, et al, Mycopathologia et Mycologia Applicata, Volume 33, pages 345—348 (1967). Chitosan is the deacylated form of chitin, which is a glucosaminoglycan obtained as a major constituent of the shells of shrimp, crabs and lobsters, the cell walls of filamentous fungi, and the exoskeletons of insects. Chitosan is commercially available in the form of fibers, for example, from Sigma-Aldrich Corporation, St. Louis, Missouri. Collagen is a fibrous protein which comprises the major portion of the white fiber in connective tissues of the animal body, particularly in the skin, bones and tendons. It is commercially available in the form of fibers, for example, from Sigma-Aldrich Corporation, St Louis, Missouri.

The water-soluble derivative of keratin employed in preparing the copolyelectrolytes of the present invention is a linear polyelectrolyte in which the keratin moiety is in anionic form. A particularly suitable anionic keratin polyelectrolyte is ammonium keratinate, obtained as the total ammonium hydroxide-soluble fraction of peracetic acid-oxidized human hair, or the alpha-keratose component of this fraction, by the procedures described in the aforementioned Rhodes, et al., article. Due to evidence indicating a higher degree of nonantigenicity, the alpha-keratose form of ammonium keratinate is preferred.

The water-soluble derivatives of the glucosaminoglycan and collagen employed in preparing the copolyelectrolytes of the present invention are linear polyelectrolytes in which the biopolymer moiety is in cationic form. Particularly suitable cationic glucosaminoglycan and collagen polyelectrolytes are the carboxylates of these biopolymers, such as their acetates or citrates, obtained by dissolving the biopolymer in an aqueous solution of the corresponding carboxylic acid. Chitosan acetate, collagen acetate, and mixtures thereof, are the preferred cationic biopolymer polyelectrolyte components.

The weight ratio of the anionic keratin polyelectrolyte component to the cationic biopolymer polyelectrolyte component in the copolyelectrolytes of the present invention, may vary over a rather wide range, and is most suitably within the range of from about 1:1 to about 10:1, and more preferably within the range of from about 2:1 to about 5:1. When the biopolymer moiety of the cationic biopolymer polyelectrolyte component is a mixture of the glucosaminoglycan and collagen, the weight ratio of the glucosaminoglycan and collagen, the weight ratio of the glucosaminoglycan and collagen, the weight ratio of the glucosaminoglycan to collagen is preferably within the range of from about 0.5:1 to about 2:1.

When the water-soluble anionic keratin polyelectrolyte component is contacted in the presence of water with the water-soluble cationic biopolymer polyelectrolyte component (i.e., cationic glucosaminoglycan, cationic collagen, or mixtures thereof), the polyelectrolyte components spontaneously rearrange themselves into a water-insoluble, water-swellable solid coherent mass. While the precise mechanism of reaction resulting in the formation of these biopolymeric copolyelectrolyte hydrogels is not known with certainty, it is believed that the initial attraction between the two polyelectrolyte components is due to their opposite net charge, and that closer juxtaposition of the biopolymer molecules then brings into play a variety of steric fitting and chemical bonding and crosslinking mechanisms at multiple sites along the molecules to produce interdigitating co-biopolymers. In any event, their properties and characteristics are totally different from those of their individual components.

In their hydrated form, the biopolymeric copolyelectrolytes of the present invention are stress-durable hydrogels which may be manipulated like a self-annealing paste or putty and thereby formed into membrane sheets, trowled into crevices, or formed in shaped containers or around glass or metal or through perforations. As long as moisture, is present, they remain flexible and elastic. As they dry down, they shrink, adhere to flat surfaces, self-anneal and harden to a crystallite form of packed microfibrils. Since they are permeable to and expansible in water, the copolyelectrolytes may be regenerated by the addition of water from their dehydrated form to their hydrogel form, and thereafter reshaped.

Preparation of the biopolymeric copolyelectrolytes of the present invention may suitably be carried out by mixing together aqueous solutions of the anionic keratin polyelectrolyte component and the cationic biopolymer polyelectrolyte component to precipitation end-point and allowing the precipitate to dry down to a cohesive membrane. The membrane may then be removed from its forming substrate either by cracking it off in its brittle dehydrated state and forming it into a powder, or by teasing and floating it off in water as a flexible hydrogel membrane.

As an alternative preparative procedure, one of the polyelectrolyte components in solid form may be contacted with an aqueous solution of the other polyelectrolyte component. For example, chitosan acetate solution alone may be dried down to a crystallite complex which resembles cellophane sheeting, its thickness and strength varying with the amount of solution used to cover a given area before dry-down. Contacting the chitosan acetate sheeting with an aqueous solution of ammonium keratinate results in the formation of a chitosan keratinate copolyelectrolyte hydrogel membrane. This could be accomplished, for example, by swabbing or spraying the ammonium keratinate solution onto a first sheet of chitosan acetate, and thereafter overlaying the wetted sheet with a second sheet of chitosan acetate.

The biopolymeric copolyelectrolytes may be formulated with various additives, such as, for example, plasticizers or softening agents, antibiotic, antifungal or other pharmaceutical agents, cells, enzymes, antibodies or pigments to enhance their properties for a particular end use. Such additives may suitably be incorporated into the copolyelectrolytes either subsequent to their formation or along with one or more of their polyelectrolyte components during their formation. When used in wound dressing applications, for example, the biopolymeric copolyelectrolytes preferably are mixed with a non-toxic plasticizer or softener, such as glycerol, in an amount sufficient to enhance the flexibility and/or adhesion of the dressing. When employing chitosan acetate sheeting as one of the polyelectrolyte components in formulating the biopolymeric copolyelectrolytes, the plasticizer or softener is advantageously incorporated into the chitosan acetate sheeting, for example, in a weight ratio of plasticizer or softener to chitosan acetate within the range of from about 0.5:1 to about 3:1.

The hydrogel membranes in accordance with the present invention may be formed in a wide range of thicknesses, the optimum thickness varying with the desired end use. Membrane thicknesses of at least about $2.54 \times 10^{-2}$ mm (about 1 mil) will have sufficient strength and durability for most applications. For membranes used as wound dressings, a thickness ranging from about $2.54 \times 10^{-2}$ mm to about $17.8 \times 10^{-2}$ mm (about 1 to about 7 mils) has been found to be particularly suitable. The membrane thickness may suitably be controlled in various ways. For example, varying the concentrations of the polyelectrolyte components in the stock solutions employed in the formation of the copolyelectrolytes will result in corresponding variations in the resulting membrane thickness. Alternatively, separately formed hydrogel layers may be laminated together, for example, with the aid of an intermediate coating of a suitable softener or plasticizer (e.g., a glycerol-water mixture), thereby forming a composite hydrogel membrane. Such composite membranes may be fabricated with their separate hydrogel layers having either the same or different composition. For example, the cationic biopolymer polyelectrolyte component of the copolyelectrolyte may be cationic chitosan in one layer and cationic collagen in another layer.

The combination of properties exhibited by the biopolymeric copolyelectrolyte hydrogel membranes of the present invention render them particularly suitable for use as dressings for burn wounds and other denuded tissue wound sites. Such membranes are rapidly, uniformly, and strongly adherent to underlying tissues by virtue of their shrinkdown from the fully hydrated state and/or by virtue of their collagen content and resulting linkage to fibrin in the wound bed. They are durable to physical stress and may be thickened as needed to enhance their durability. They have a high degree of absorbancy for serous or bloody exudate, and remain flexible and elastic so long as moisture is present. Their water vapor transport characteristics are such as to allow pervaporation of water at a rate sufficiently high so as to prevent fluid pooling beneath the dressing, and yet sufficiently low so as to maintain the requisite moisture at the wound surface for wound healing cell migration to occur and the requisite moisture within the membrane for maintenance of flexibility and elasticity. The hydrogel membranes are highly permeable to oxygen, allowing air to get into the wound while stopping bacteria. Their microbial barrier function can be further improved by incorporating antimicrobial agents into the dressing, for example, by inclusion in the hydrogel during its formation, by inclusion between hydrogel layers, or by direct spraying or smearing onto the wound dressing as clinical conditions demand.

The materials used in making the copolyelectrolytes and their hydrogel membranes are non-antigenic and non-toxic, and are readily available. Furthermore, the copolyelectrolytes, either in dehydrated or hydrated form, are easily storable at room temperature in polyethylene bags, aluminum foil packs, or plastic dishes after autoclaving, gas, alcohol, or radiation sterilization.

The copolyelectrolyte hydrogel membrane wound dressings may be readily and conveniently applied to the wound site in several different application modes. For example, the hydrogel may be preformed and applied to the wound site either as a preformed membrane or as a self-annealing paste. Alternatively, the copolyelectrolyte may be preformed and applied to the wound site in dehydrated form, either as a dried membrane or as a powder, and thereafter allowed to hydrate to an adherent conforming hydrogel membrane in situ on the wound site. A further alternative application mode is to individually apply the anionic keratin polyelectrolyte component and the cationic biopolymer polyelectrolyte component to the wound site so as to effect in situ formation on the wound site of the copolyelectrolyte as a hydrogel membrane. In this latter mode of application, both of the polyelectrolyte components may be applied as aqueous solutions, or one may be applied as an aqueous solution and the other in solid form. For example, the wound site may be first sprayed or swabbed with an aqueous solution of ammonium keratinate, and thereafter overlayed with dried chitosan acetate sheeting, preferably including a flexibility — and/ or adhesion-enhancing amount of a non-toxic plasticizer or softener, such as glycerol.

The copolyelectrolyte hydrogel membrane wound coverings can remain in place over substantially the entire wound healing period, during which time fluid exudate from the wound is absorbed, and white blood cells and macrophages infiltrate the hydrogel and ultimately dry at the upper air interface to form a protective scab, while entering fibroblasts elaborating collagen fibers bind to the lower moist surface of the membrane. Epidermal cells move in centripetally from the edge of the wound through these collagen fibers to grow over and close the wound in the moist space between the fibroblasts and the white cell-infiltrated membrane. The moist membrane becomes biodegraded by the skin cells, white cells and macrophages. In the latter stages of wound healing, when moisture through the wound area is much reduced, the remaining membrane will dry and harden to a protective carapace or scab, which will fall off naturally without leaving any scarring. The dried scab may, if desired, be softened and removed by application of a glycerine-water mixture.

While the hydrophilic biopolymeric copolyelectrolytes of the present invention have been described primarily with reference to their utility as hydrogel membrane wound dressings, it will be understood that these novel materials have a wide variety of other potentially important applications. For example, they may be used in conjunction with implantable prosthetic devices, and as scar tissue coverings, sutures, tapes, sustained release drug carriers, and tube lining in bypass surgery.

The invention is further illustrated by way of the following examples.

## Example 1

Stock solutions for use in preparing hydrophilic biopolymeric copolyelectrolytes in accordance with the present invention were prepared in the following manner.

Chitosan acetate solution was prepared by continuously stirring 50 mg of practical grade shrimp chitosan fibers (Sigma-Aldrich Corporation, St. Louis, Missouri) into 100 ml of cold 0.25% (v/v) acetic acid. The solution was cleared of gross particulate matter by filtration through a 12-layer gauze pad on a Buchner funnel.

Collagen acetate solution was prepared by stirring 200 mg of bovine collagen fibers (acid-soluble Type III, Sigma-Aldrich Corporation, St. Louis, Missouri) in 100 ml of cold 0.25% (v/v) acetic acid.

Alpha-keratose ammonium keratinate solution was prepared as follows. 12 grams of clean, dry, blond human hair, previously degreased and washed, were placed in a 1 liter Erlynmeyer flask containing 320 ml of water. 80 ml of concentrated peracetic acid was added, and the flask stoppered. The contents of the flask were swirled and then placed in a refrigerator for 24 hours with occasional swirling. The bleached, easily stretched and torn ("retted") hair was then freed of the peracetic acid by decantation and thorough washing with separate water rinses. The washed retted hair was then covered with 800 ml of 3 N ammonium hydroxide, and stirred in the cold for 24 hours to solubilize hair keratins. The total soluble protein (TP) fraction was then cleared of solids by centrifugation, discarding undissolved protein and non-protein residual debris. The TP fraction was further clarified through Whatman No. 1 paper, dialyzed against water until the dialyzate wash gave only a faint positive for ammonia with Nessler's reagent, and then Seitz microfiltered. Alpha-keratose was precipitated from the TP fraction by incremental addition of 0.1 N hydrochloric acid while stirring. The precipitate was collected by centrifugation, and the supernatant gamma keratose, antigenic for rabbits, was discarded. The alpha-keratose precipitate was washed in water, re-centrifuged, and then solubilized in 0.1 N ammonium hydroxide. A second cycle of precipitation, washing, and solubilization was run on the alpha-keratose before final dialysis against water, concentraton of the protein microfiltration, and storage in a sterile container. The resulting solution contained approximately 7.5 mg of alpha-keratose ammonium keratinate per ml.

## Example 2

The collagen acetate and alpha-keratose ammonium keratinate stock solutions prepared in Example 1, were employed in the preparation of a collagen keratinate copolyelectrolyte. 10 ml of the collagen acetate solution (containing 20 mg of collagen acetate) and 10 ml of the ammonium keratinate solution (containing 75 mg of alpha-keratose ammonium keratinate) were mixed together in a plastic Petri dish to precipitation endpoint. Upon evaporation of the supernatant, the precipitate dried down into a dry crystalline-like

sheet. Upon adding water to the dried down precipitate, a self-annealed flexuous, diaphanous, cohesive collagen keratinate copolyelectrolyte hydrogel membrane was formed, which was teased and floated from the bottom of the dish.

### Example 3

The chitosan acetate and alpha-keratose ammonium keratinate stock solutions prepared in Example 1, were employed in the preparation of a chitosan keratinate copolyelectrolyte. 50 ml of the chitosan acetate solution (containing 25 mg of chitosan acetate) and 15 ml of the ammonium keratinate solution (containing 112.5 mg of alpha-keratose ammonium keratinate), were mixed together in a plastic Petri dish to precipitation end-point to produce an opaque whitish, sticky, flocculent precipitate. Upon evaporation of the supernatant, the precipitate dried down to form a hard brittle translucent sheet, which was pried or cracked from the bottom of the dish. Upon adding water to the dried material, a self-annealed, tough, stretchable, cuttable, cohesive chitosan keratinate copolyelectrolyte hydrogel membrane was formed.

### Example 4

All three of the stock solutions prepared in Example 1, were employed in the preparation of a chitosan-collagen keratinate copolyelectrolyte of a chitosan-collagen keratinate copolyelectrolyte. 30 ml of the chitosan acetate solution (containing 15 mg of chitosan acetate), 10 ml of the collagen acetate solution (containing 20 mg of collagen acetate), and 10 ml of the ammonium keratinate solution (containing 75 mg of alpha-keratose ammonium keratinate), were mixed together in a plastic Petri dish to precipitation end-point. Upon evaporation of the supernatant, the precipitate dried down to a crystalline-like sheet. Upon adding water to the dried down precipitate, a self-annealed, flexible, cohesive chitosan-collagen keratinate copolyelectrolyte hydrogel membrane was formed, which was teased and floated from the bottom of the dish.

### Example 5

This example illustrates the preparation of a chitosan keratinate copolyelectrolyte hydrogel membrane employing solid chitosan acetate sheeting as the cationic chitosan polyelectrolyte component.

Chitosan acetate sheeting was prepared by mixing together 2 ml of glacial acetic acid, 4 ml of glycerol, 794 ml of water, and 4 g of practical grade shrimp chitosan fibers (Sigma-Aldrich Corporation, St. Louis, Missouri). The mixture was stirred until a solution was formed. The solution was filtered through five layers of cheese cloth, poured into a flat pan, an allowed to dry down to form a flexible, sticky, cohesive solid sheet of chitosan acetate.

When the chitosan acetate sheeting was contacted with the alpha-keratose ammonium keratinate stock solution prepared in Example 1, the solid sheeting swelled to form a self-annealed, flexible, cohesive chitosan keratinate copolyelectrolyte hydrogel membrane.

### Example 6

The ears of a ketamine anaesthetized 10 pound male New Zealand white rabbit were shorn of hair and prepared for surgery. From the dorsal surface of one ear, a full thickness circle of skin 2.5 cm in diameter was removed, and the wound sponged dry. Sterile alpha-keratose ammonium keratinate solution was dropped into the wound area and on the surrounding shaven skin. A circular swatch of thin chitosan acetate sheeting (2.4 mg chitosan/cm$^2$) was fitted over the wound area and surrounding skin, resulting in the formation of a chitosan keratinate copolyelectrolyte hydrogel membrane wound dressing, which became tightly bound to all surfaces in a few minutes of drying time. The area was dressed with sterile petrolatum gauze, bandaged and taped. A control ear was also prepared, wherein the wound was dressed only with sterile petrolatum gauze, bandaged and taped.

Examination of the test ear at 10 days postoperation (P.O.) showed a flat scab with normal healing, absorption and disappearance of the copolyelectrolyte hydrogel membrane. The control ear showed normal healing with a central heaped scab. At 14 days P.O., only small residual scabs remained on both ears. During periodic examinations, gauze dressing adherent to the copolyelectrolyte hydrogrel membrane was easily separated with a simple saline wash.

### Example 7

A 10 pound female rabbit was prepared for surgery and a 3.5 cm diameter full thickness of skin was removed from the left flank. The site was then sprayed with sterile alpha-keratose ammonion keratinate solution, and covered with a medium weight chitosan acetate sheet (3.6 mg chitosan/cm$^2$), dressed with petrolatum gauze, bandaged, and taped.

Examinations at 3, 7, and 10 days P.O. showed no remarkable changes over the normal healing process. Formation of a capillary net and peripheral ingrowth of new tissue could be observed through the copolyelectrolyte hydrogel membrane "window". Hydrogel membrane overlapping the surrounding normal skin also remained pliable and adherent. Between 10 and 17 days P.O., the lesion had shrunk to 1.9 cm in diameter with the hydrogel membrane absorbed and visible only on edge, sandwiched between the new tissue in the wound area below and a dry scab above. On day 20 P.O., the lesion was further reduced to 1.7 cm in diameter, at which point the experiment was terminated in order to examine the wound site histologically and cytochemically before all the copolyelectrolyte hydrogel membrane was completely absorbed.

### Example 8

A 10 pound female rabbit was prepared for surgery, and a full thickness of skin removed from a rectangular area 2.5 × 3.5 cm². After spraying the wound area with sterile alpha-keratose ammonium keratinate solution, a double layer of chitosan acetate sheeting (two sheets annealed with alpha-keratose ammonium keratinate solution and containing a total of 6.4 mg chitosan/cm²) was applied to the wound and surrounding skin. The area was then dressed with sterile petrolatum gauze, bandaged, and taped.

The copolyelectrolyte hydrogel membrane remained flexible over the moist wound site for 14 days. In drying and contracting over the surrounding skin, the membrane caused puckering, which was relieved by application of a glycerine-water (1:1) solution. Accelerated wound closure with regrowth of fur took place between days 17 and 27 P.O., the area undergoing repair closing to 2 × 3 cm. The remaining flat membrane surface resembled a hard shell adherent scab under which repair was proceeding. Further bandaging and taping were eliminated as unnecessary for care of the site, and healing was complete 30 days P.O.

### Example 9

A rabbit was prepared for surgery and a full thickness of skin removed from an approximately square area 10 cm × 10 cm (4 inches × 4 inches). After spraying the area with alpha-keratose ammonium keratinate solution, a double-layered chitosan acetate sheeting similar to that employed in Example 8, was applied to the wound and surrounding skin. The area was then dressed with sterile petrolatum gauze, bandaged, and taped. A control wound of the same approximate size was also prepared, and was dressed only with sterile petrolatum gauze, bandaged, and taped. The wound sites were periodically examined for wound closure. During the first three weeks P.O., wound closure proceeded in the copolyelectrolyte hydrogel membrane-covered wound at a 50% faster rate than in the control wound.

### Example 10

A goat was prepared for surgery, and full thicknesses of skin were removed from a rectangular area 20.3 cm × 22.9 cm (8 inches × 9 inches) on one flank and from a rectangular area 17.8 cm × 20.3 cm (7 inches × 8 inches) on the other flank. The smaller size wound was used as the control, and was dressed only with sterile petrolatum gauze, bandaged, and taped. The larger size wound was sprayed with sterile alpha-keratose ammonium keratinate solution, overlayed with a double layered chitosan acetate sheeting similar to that employed in Example 8, and then dressed with sterile petrolatum gauze, bandaged, and taped. The two wounds were examined periodically for wound healing and closure. After four days P.O., the copolyelectrolyte hydrogel membrane-covered wound was completely covered with fibroblasts, whereas the control wound showed no signs of healing. After 14 days P.O., the copolyelectrolyte hydrogel membrane-covered wound had closed 5.1 cm (2 inches), while the control wound had closed less than 2.55 cm (1 inch). After 66 days P.O., the hydrogel membrane-covered wound had been reduced to 1 inch × 3 inches; whereas after 78 days P.O., the control wound had only closed 5.1 cm × 10.2 cm (2 inches × 4 inches).

### Claims

1. A hydrophilic biopolymeric copolyelectrolyte of (a) a water-soluble linear anionic protein polyelectrolyte component derived from keratin, and (b) a water-soluble linear cationic biopolymer polyelectrolyte component derived from at least one biopolymer selected from a glucosaminoglycan and collagen.

2. The copolyelectrolyte of Claim 1, wherein said anionic protein polyelectrolyte component is ammonium keratinate.

3. The copolyelectrolyte of Claim 2, wherein the keratin moiety of said ammonium keratinate is alphakeratose.

4. The copolyelectrolyte of any preceding claim, wherein said glucosaminoglycan is chitosan.

5. The copolyelectrolyte of any preceding claim, wherein said cationic biopolymer polyelectrolyte component is a biopolymer carboxylate.

6. The copolyelectrolyte of Claim 5, wherein said biopolymer carboxylate is a biopolymer acetate.

7. The copolyelectrolyte of Claim 1, wherein the weight ratio of said anionic protein polyelectrolyte component to said cationic biopolymer polyelectrolyte component is within the range of from about 1:1 to about 10:1.

8. The copolyelectrolyte of Claim 7, wherein the weight ratio of said anionic protein polyelectrolyte component to said cationic biopolymer polyelectrolyte component is within the range of from about 2:1 to about 5:1.

9. The copolyelectrolyte of Claim 8, wherein the biopolymer moiety of said cationic biopolymer polyelectrolyte component is a mixture of chitosan and collagen in a chitosan to collagen weight ratio of from about 0.5:1 to about 2:1.

10. The copolyelectrolyte of Claim 9, wherein said anionic protein polyelectrolyte component is ammonium keratinate, and said cationic biopolymer polyelectrolyte component is a mixture of chitosan acetate and collagen acetate.

11. The copolyelectrolyte of Claim 8, wherein said anionic protein polyelectrolyte component is ammonium keratinate, and said cationic biopolymer polyelectrolyte component is either chitosan acetate or collagen acetate.

12. The copolyelectrolyte of Claim 10 or Claim 11, wherein the keratin moiety of said ammonium keratinate is alpha-keratose.

13. A composition comprising the copolyelectrolyte of any preceding claim, and a flexibility — and/or adhesion-enhancing amount of a nontoxic plasticizer or softener.

14. The composition of Claim 13, wherein said plasticizer or softener is glycerol.

15. A hydrogel comprising the copolyelectrolyte of any one of Claims 1—12 in hydrated form.

16. A membrane comprising at least one layer of the hydrogel of Claim 15, said membrane having a thickness of at least about 2.54 × $10^{-2}$ mm.

17. The membrane of Claim 16, wherein both cationic chitosan and cationic collagen are present either in the same hydrogel layer or in two separate hydrogel layers.

18. A biodegradable dressing for burn wounds and other denuded tissue wound sites, comprising the membrane of Claim 16 or Claim 17.

19. The dressing of Claim 18, wherein said membrane includes a flexibility — and/or adhesion-enhancing amount of a non-toxic plasticizer or softener.

20. A composition comprising the copolyelectrolyte of any one of Claims 1—12 in powder form.

21. A process for preparing a hydrophilic biopolymeric copolyelectrolyte, which process is characterised by contacting reactant (a) with reactant (b) in the presence of water, reactant (a) being a water-soluble linear cationic biopolymer polyelectrolyte component derived from at least one biopolymer selected from a glucosaminoglycan and collagen.

22. A process according to Claim 21, wherein said reactant (a) and/or said reactant (b) are as defined in any one of Claims 2—12.

23. A method of preparing a biodegradable dressing for burn wounds and other denuded tissue wound sites, comprising forming a membrane of at least one layer of a hydrophilic biopolymeric copolyelectrolyte including (a) a water-soluble linear anionic protein polyelectrolyte component derived from keratin, and (b) a water-soluble linear cationic biopolymer polyelectrolyte component derived from at least one biopolymer selected from a glucosaminoglycan and collagen.

24. The method of Claim 23, wherein a solution of said anionic protein polyelectrolyte component and a solution of said cationic biopolymer polyelectrolyte component are mixed together and the resulting mixture is allowed to dry down to form said membrane.

25. The method of Claim 23, comprising preparing a membrane of a cationic biopolymer polyelectrolyte component and spraying said membrane with a solution of an anionic protein polyelectrolyte component.

26. The method of Claim 23, further comprising the step of dehydrating said copolyelectrolyte membrane.

27. The method of Claim 26, further comprising the step of reducing said dehydrated copolyelectrolyte membrane to a powder form.

28. The method of Claim 27, wherein said powder dehydrated copolyelectrolyte membrane is rehydrated prior to use.

29. The method of Claim 27, wherein said dehydrated copolyelectrolyte membrane in powder form is allowed to rehydrate in situ at a wound site.

**Patentansprüche**

1. Ein hydrophiler, biopolymerer Copolyelektrolyt aus (a) einem wasserlöslichen, linearen, anionischen Protein-Polyelektrolytbestandteil, der von Keratin abgeleitet ist, und (b) einem wasserlöslichen, linearen, katinischen Biopolymer-Polyelektrolytbestandteil, der von wenigstens einem Biopolymer abgeleitet ist, das aus einem Glucosaminoglycan und Collagen ausgewählt ist.

2. Der Copolyelektrolyt nach Anspruch 1, worin der genannte anionische Protein-Polyelektrolytbestandteil Ammoniumkeratinat ist.

3. Der Copolyelektrolyt nach Anspruch 2, worin der Keratinanteil des genannten Ammoniumkeratinats Alphakeratose ist.

4. Der Copolyelektrolyt nach einem der vorhergehenden Ansprüche, worin das genannte Glucoseaminoglycan Chitosan ist.

5. Der Copolyelektrolyt nach einem der vorhergehenden Ansprüche, worin der genannte kationische Biopolymer-Polyelektrolytbestandteil ein Biopolymercarboxylat ist.

6. Der Copolyelektrolyt nach Anspruch 5, worin das genannte Biopolymercarboxylat ein Biopolymeracetat ist.

7. Der Copolyelektrolyt nach Anspruch 1, worin das Gewichtsverhältnis des genannten anionischen Protein-Polyelektrolytbestandteils zum genannten kationischen Biopolymer-Polyelektrolytbestandteil innerhalb eines Bereiches von etwa 1:1 zu etwa 10:1 liegt.

8. Der Copolyelektrolyt nach Anspruch 7, worin das Gewichtsverhältnis des genannten anionischen Protein-Polyelektrolytbestandteils zum genannten kationischen Biopolymer-Polyelektrolytbestandteil innerhalb eines Bereiches von etwa 2:1 zu etwa 5:1 liegt.

9. Der Copolyelektrolyt nach Anspruch 8, worin der Biopolymer-Anteil des genannten kationischen Biopolymer-Polyelektrolytbestandteils ein Gemisch aus Chitosan und Collagen in einem Gewichtsverhältnis von Chitosan zu Collagen von etwa 0,5:1 bis etwa 2:1 ist.

10. Der Copolyelektrolyt nach Anspruch 9, worin der genannte anionische Protein-Polyelektrolytbestandteil Ammoniumkeratinat ist und der genannte kationische Biopolymer-Polyelektrolytbestandteil ein Gemisch aus Chitosanacetat und Collagenacetat ist.

11. Der Copolyelektrolyt nach Anspruch 8, worin der genannte anionische Protein-Polyelektrolytbestandteil Ammoniumkeratinat und der genannte kationische Biopolymer-Polyelektrolytbestandteil entweder Chitosanacetat oder Collagenacetat ist.

12. Der Copolyelektrolyt nach Anspruch 10 oder 11, worin der Keratinanteil des genannten Ammoniumkeratinats Alphakeratose ist.

13. Eine Zusammensetzung umfassend den Copolyelektrolyten nach einem der vorhergehenden Ansprüche, und eine flexibilitäts- und/oder adhäsionsverstärkende Menge eines nichttoxischen Plastifikators oder Weichmachers.

14. Die Zusammensetzung nach Anspruch 13, worin der genannte Plastifikator oder Weichmacher Glyzerin ist.

15. Ein Hydrogel umfassend den Copolyelektrolyten nach einem der Ansprüche 1 bis 12 in hydratisierter Form.

16. Eine Membran umfassend wenigstens eine Schicht des Hydrogels nach Anspruch 15, wobei die genannte Membran eine Dicke von wenigstens etwa $2,54 \times 10^{-2}$ mm aufweist.

17. Die Membran nach Anspruch 16, worin sowohl kationisches Chitosan als auch kationisches Collagen entweder in der gleichen Hydrogelschicht oder in zwei getrennten Hydrogelschichten vorliegen.

18. Ein biologisch abbaubarer Wundverband für Brandwunden und andere Wundstellen mit freigelegtem Gewebe umfassend die Membran nach Anspruch 16 oder 17.

19. Der Wundverband nach Anspruch 18, worin die genannte Membran eine flexibilitäts- und/oder adhäsionsverstärkende Menge eines nicht toxischen Plastifikators oder Weichmachers einschließt.

20. Eine Zusammensetzung umfassend den Copolyelektrolyten nach einem der Ansprüche 1 bis 12 in Pulverform.

21. Ein Verfahren zur Herstellung eines hydrophilen, biopolymeren Copolyelektrolyten, welches Verfahren dadurch gekennzeichnet ist, daß der Reaktionspartner (a) mit dem Reaktionspartner (b) in Gegenwart von Wasser in Berührung gebracht wird, und daß der Reaktionspartner (a) ein wasserlöslicher, linearer, anionischer Protein-Polyelektrolytbestandteil ist, der von Keratin abgeleitet ist, und der Reaktionspartner (b) ein wasserlöslicher, linearer, kationischer Biopolymer-Polyelektrolytbestandteil ist, der aus wenigstens einem Biopolymer abgeleitet ist, das aus einem Glucosaminoglycan und Collagen ausgewählt ist.

22. Ein Verfahren nach Anspruch 21, worin der genannte Reaktionspartner (a) und/oder der genannte Reaktionspartner (b) wie in einem der Ansprüche 1 bis 12 definiert ist.

23. Ein Verfahren zur Herstellung eines biologisch abbaubaren Wundverbandes für Brandwunden und andere Wundstellen mit freigelegtem Gewebe, umfassend die Bildung einer Membran aus wenigstens einer Schicht eines hydrophilen, biopolymeren Copolyelektrolyten beinhaltend (a) einen wasserlöslichen, linearen, anionschen Protein-Polyelektrolytbestandteil, der von Keratin abgeleitet ist und (b) einen wasserlöslichen, linearen, kationischen Biopolymer-Polyelektrolytbestandteil , der aus wenigstens einem Biopolymer abgeleitet ist, das aus einem Glucosaminoglycan und Collagen ausgewählt ist.

24. Das Verfahren nach Anspruch 23, worin eine Lösung aus dem genannten anionischen Protein-Polyelektrolytbestandteil und eine Lösung aus dem genannten kationischen Biopolymer-Polyelektrolytbestandteil zusammengemischt werden und die entstandene Mischung trocknen gelassen wird, um die genannte Membran zu formen.

25. Das Verfahren nach Anspruch 23, umfassend die Herstellung einer Membran aus einem kationischen Biopolymer-Polyelektrolytbestandteil und Besprühen der genannten Membran mit einer Lösung aus einem anionischen Protein-Polyelektrolytbestandteil.

26. Das Verfahren nach Anspruch 23, weiters umfasend den Schritt des Dehydratisierens der genannten Copolyelektrolytmembran.

27. Das Verfahren nach Anspruch 26, weiters umfassend den Schritt der Überführung der genannten dehydratisierten Copolyelektrolytmembran in Pulverform.

28. Das Verfahren nach Anspruch 27, worin die genannte dehydratisierte, pulverförmige Copolyelektrolytmembran vor der Verwendung rehydratisiert wird.

29. Das Verfahren nach Anspruch 27, worin die genannte pulverförmige dehydratisierte Copolyelektrolytmembran an Ort und Stelle auf einer Wundstelle rehydratisieren gelassen wird.

**Revendications**

1. Copolyélectrolyte biopolymère hydrophile comportant (a) un composant polyélectrolyte de protéine anionique linéaire hydrosoluble provenant de la kératine et (b) un composant polyélectrolyte biopolymère cationique linéaire hydrosoluble provenant d'au moins un biopolymère choisi parmi un glucosaminoglycanne et du collagène.

2. Copolyélectrolyte selon la revendication 1, dans lequel ledit composant polyélectrolyte de protéine anionique est du kératinate d'ammonium.

3. Copolyélectrolyte de la revendication 2, dans lequel le fragment kératine du kératinate d'ammonium est de l'alpha-kératose.

4. Copolyélectrolyte de l'une quelconque des revendications précédentes, dans lequel ledit glucosamino-glycanne est du chitosane.

5. Copolyélectrolyte selon l'une quelconque des revendications précédentes, dans lequel ledit composant polyélectrolyte biopolymère cationique est un carboxylate de biopolymère.

6. Copolyélectrolyte de la revendication 5, dans lequel ledit carboxylate de biopolymère est un acétate de biopolymère.

7. Copolyélectrolyte de la revendication 1, dans lequel le rapport pondéral du composant polyélectrolyte de type protéine anionique audit composant polyélectrolyte biopolymère cationique se situe dans l'intervalle compris entre environ 1:1 et environ 10:1.

8. Copolyélectrolyte de la revendication 7, dans lequel le rapport pondéral dudit composant polyélectrolyte de type protéine anionique audit composant polyélectrolyte biopolymère cationique se situe dans l'intervalle compris entre environ 2:1 et environ 5:1.

9. Copolyélectrolyte de la revendication 8, dans lequel le fragment biopolymère dudit composant polyélectrolyte biopolymère cationique est un mélange de chitosane et de collagène selon un rapport pondéral du chitosane au collagène compris entre environ 0,5:1 et environ 2:1.

10. Copolyélectrolyte de la revendication 9, dans lequel ledit composant polyélectrolyte de type proteine anionique est du kérantinate d'ammonium, et ledit composant polyélectrolyte biopolymère cationique est un mélange d'acétate de chitosane et d'acétate de collagène.

11. Copolyélectrolyte de la revendication 8, dans lequel ledit composant polyélectrolyte de type protéine anionique est du kératinate d'ammonium, et ledit composant polyélectrolyte biopolymère cationique est de l'acétate de chitosane ou de l'acétate de collagène.

12. Copolyélectrolyte de la revendication 10 ou de la revendication 11, dans lequel le fragment kératine dudit kératinate d'ammonium est de l'alpha-kératose.

13. Composition comprenant le copolyélectrolyte de n'importe quelle revendication précédente, et une quantité, capable d'amplifier la flexibilité et/ou l'adhérence, d'un plastifiant ou assouplissant non toxiques.

14. Composition selon la revendication 13, dans laquelle ledit plastifiant ou assouplissant est du glycérol.

15. Hydrogel comprenant le copolyélectrolyte de l'une quelconque des revendications 1 à 12 sous forme hydratée.

16. Membrane comprenant au moins une couche de l'hydrogel de la revendication 15, ladite membrane ayant une épaisseur d'au moins 2,54 $\times$ 10$^{-2}$ mm environ.

17. Membrane de la revendication 16, dans laquelle du chitosane cationique et du collagène cationique sont tous deux présents dans la même couche d'hydrogel ou dans deux couches séparées d'hydrogel.

18. Pansement biodégradable pour brûlures ou autres sites de plaies à tissus dénudés, comprenant la membrane de la revendication 16 ou de la revendication 17.

19. Pansement de la revendication 18, dans lequel ladite membrane comprend une quantité, capable d'amplifier la flexibilité et/ou l'adhérence, d'un plastifiant ou assouplissant non-toxique.

20. Composition comprenant la copolyélectrolyte de l'une quelconque des revendications 1 à 12, sous forme de poudre.

21. Procédé pour préparer un copolyélectrolyte biopolymère hydrophile, ce procédé étant caractérisé en ce qu'on met en contact un matière (a) destinée à réagir avec une matière (b) destinée à réagir, en présence d'eau, la matière (a) étant un composant polyélectrolyte de type protéine anionique linéaire hydrosoluble provenant de la kératine, et la matière (b) étant un composant polyélectrolyte biopolymère cationique linéaire hydrosoluble provenant d'au moins un biopolymère choisi parmi un glucosaminoglycanne et du collagène.

22. Procédé selon la revendication 21, dans lequel ladite matière (a) et/ou ladite matière (b) destinée(s) à réagir sont comme définies dans l'une quelconque des revendications 2 à 12.

23. Procédé pour préparer un pansement biodégradable pour brûlures et autres sites de plaies à tissus dénudés, comprenant la formation d'une membrane d'au moins une couche d'un copolyélectrolyte biopolymère hydrophile comprenant (a) un composant polyélectrolyte de type proteine anionique linéaire hydrosoluble provenant de la kératine et (b) un composant polyélectrolyte biopolymère cationique linéaire hydrosoluble provenant d'au moins un biopolymère choisi parmi un glucosaminoglycanne et du collagène.

24. Procédé de la revendication 23, dans lequel on mélange ensemble une solution dudit composant polyélectrolyte de type protéine anionique et une solution dudit composant polyélectrolyte biopolymère cationique, et on laisse le mélange résultant sécher pour former ladite membrane.

25. Procédé de la revendication 23, comprenant la préparation d'une membrane d'un composant polyélectrolyte biopolymère cationique et la projection de pulvérisation, sur ladite membrane, d'une solution d'un composant polyélectrolyte de type protéine anionique.

26. Procédé de la revendication 23, comprenant en outre l'étape consistant à déshydrater ladite membrane de copolyélectrolyte.

27. Procédé de la revendication 26, comprenant en outre l'étape consistant à réduire sous forme de poudre ladite membrane de copolyélectrolyte déshydratée.

28. Procédé de la revendication 27, dans lequel on réhydrate avant utilisation ladite membrane de copolyélectrolyte déshydratée en poudre.

29. Procédé selon la revendication 27, dans lequel on laisse se réhydrater "in situ", au site de la plaie, ladite membrane de copolyélectrolyte déshydratée sous forme de poudre.